# EUROPÄISCHE PATENTSCHRIFT

(11) **EP 2 186 487 B1**
(45) Veröffentlichungstag und Bekanntmachung des Hinweises auf die Patenterteilung: **18.01.2012**
(21) Anmeldenummer: 09014336.3
(22) Anmeldetag: 17.11.2009
(51) Int. Cl.: A61B 17/16, A61B 17/29

(54) **Chirurgisches Instrument mit Getriebe**
Surgical instrument with gearbox
Instrument chirurgical doté d'un engrenage

(30) Priorität: 18.11.2008 DE 202008015256 U
(43) Veröffentlichungstag der Anmeldung: 19.05.2010
(73) Patentinhaber: Wenzler & Co., 78582 Balgheim (DE)
(72) Erfinder: Daumüller, Thomas, 70771 Leinfelden-Echterdingen (DE)
(74) Vertreter: Modrow, Stephanie

(56) Entgegenhaltungen:
- DE-B1- 2 808 911
- US-A- 5 465 895
- US-A- 5 653 713
- US-A- 5 792 074
- US-A1- 2008 237 298
- US-B1- 6 699 255

## Beschreibung

Die Erfindung bezieht sich auf ein chirurgisches Instrument, welches es dem Chirurgen ermöglicht, mit dem Schliessen bzw. Öffnen seiner Hand, einen grossen Arbeitsbeweg umzusetzen.

### Stand der Technik

Es sind chirurgische Instrumente bekannt, die über Hebelverhältnisse oder Verzahnungen geöffnet und geschlossen werden können. Das Problem dieser Instrumente liegt darin, dass von den Chirurgen eine möglichst grosse Öffnung der Instrumente verlangt wird, diese aber durch den Weg der Hand und die Hebelverhältnisse der Instrumente stark eingeschränkt sind.

Als Stand der Technik werden die US 5,792,074 A, DE 28 08 911 B1 und US 6,669,255 B1 genannt.

### Aufgabenstellung

Der Erfindung liegt die Aufgabe zugrunde, eine möglichst große Öffnung des Instrumentes zu erreichen. Dies wird mittels einer Getriebeübersetzung erzielt, die nachfolgend beschrieben wird.

### Ausführungsbeispiel

Ein Ausführungsbeispiel der Erfindung wird anhand der Zeichnungen erläutert.

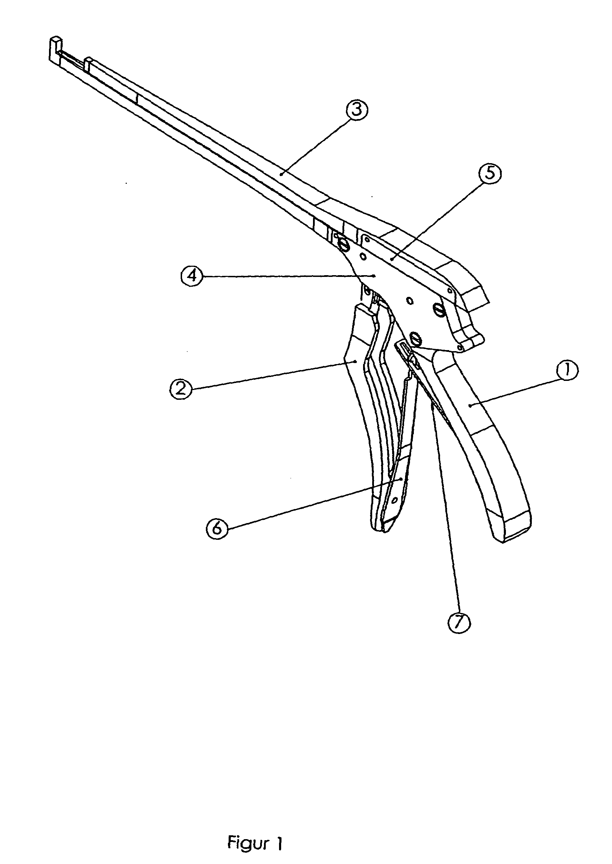

Die Fig. 1 zeigt das chirurgische Instrument mit dem Griffstück 1 und dem Hebel 2, die über die Federn 6 und 7 maximal so weit gespreizt werden, dass man sie mit der menschlichen Hand noch umschließen kann.

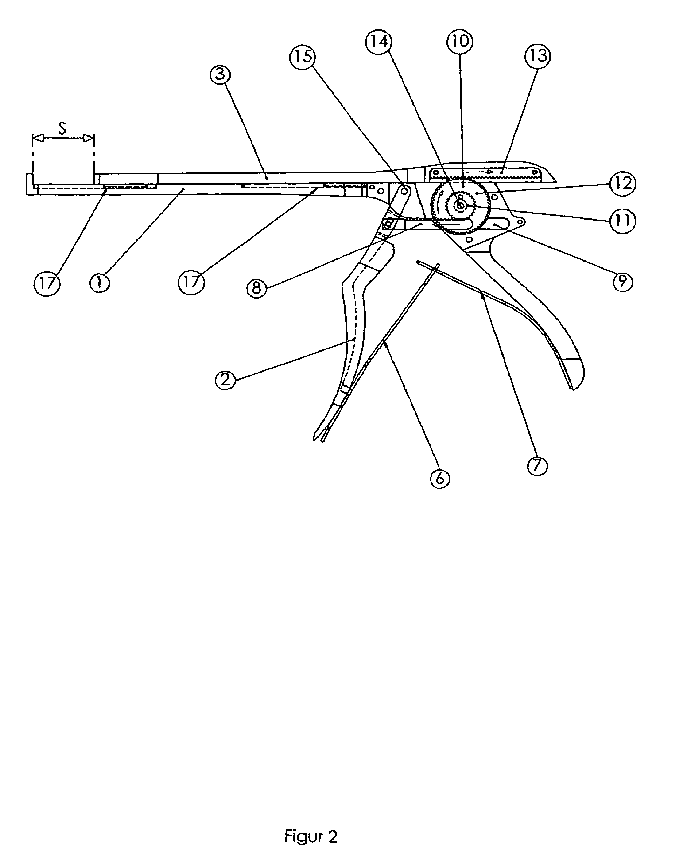

Der Deckel 4 schützt das sich im Griffstück 1 befindende Getriebe, über welches der Schieber 3 beweget werden kann. Der Deckel 4 führt die in der folgenden Fig. 2 näher beschriebene Zahnstange A 8 seitlich.

Die Blende 5 verdeckt die Zahnstange B 13, die mit dem Schieber 3 fest verbunden ist.

Fig. 2 zeigt das chirurgische Instrument ohne den Deckel 4 und die Blende 5 in maximal geöffnetem Zustand. Der mit S gekennzeichnete Doppelpfeil zeigt diesen Abstand von Griffstück 1 und Schieber 3. Der Abstand entspricht dem Arbeitsweg des Instrumentes.

Der Hebel 2 ist über die Hebelachse 15 im Griffstück 1 drehbar gelagert. Das Griffstück 1 hat eine Ausnehmung 9, in welcher die Zahnstange A 8 linear geführt wird. Im Hebel 2 ist ein Langloch 16 ausgespart. Die Zahnstange A 8 hat einen Zapfen, der im Langloch 16 des Hebels 2 mitgenommen wird.

Die Erfindung zeichnet sich dadurch aus, dass im Griffstück 1 ein Zahnradpaar 10 drehbar gelagert ist. Das Zahnradpaar 10 besteht aus dem Zahnrad 11 und dem damit fest verbundenen Zahnrad 12. Die beiden Zahnräder 11 und 12 sind in ihrer Mittelachse koaxial gelagert. Die Zahnstange A 8 greift in das Zahnrad 11 ein.

Die Federn 6 und 7 öffnen mit ihrer Federkraft den Winkel zwischen Hebel 2 und Griffstück 1 so weit wie möglich. Die Zahnstange A 8 bewegt sich dabei linear, da in der Ausnehmung 9 geführt, mit dem Hebel 2. Das Zahnradpaar 10 wird durch die Linearbewegung der Zahnstange A 8 gedreht, und schiebt ihrerseits über die Zahnstange B 13 den Schieber 3 in gegenläufiger Richtung zur Bewegung der Zahnstange A 8. Die Führungen 17 können als T-Nutenführungen, Schwalbenschwanzführungen o. ä. ausgebildet sein. Sie sorgen dafür, dass Griffstück 1 und Schieber 3 parallel gleitend miteinander verbunden sind.

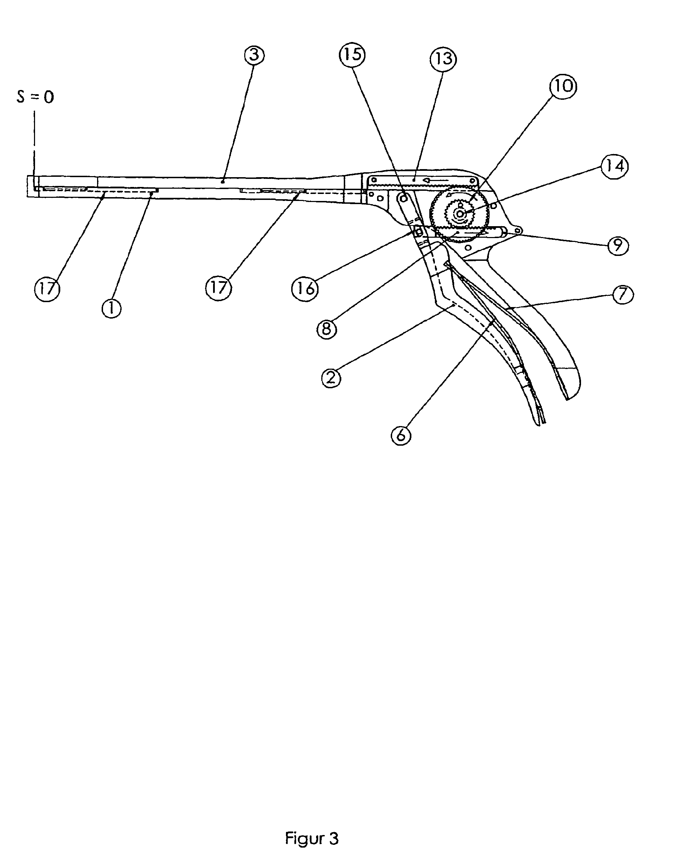

In Fig. 3 wird das Schließen des Instrumentes aufgezeigt.

Die Hand des Chirurgen bewegt den Hebel 2, der im Griffstück 1 über die Hebelachse 15 drehbar gelagert ist, entgegen der Federkräfte der Federn 6 und 7.

Die Zahnstange A 8 wird dabei linear in das Griffstück 1 geschoben. Die Bewegungsrichtung ist durch den Pfeil auf der Zahnstange A 8 gekennzeichnet. Da die Zahnstange A 8 in das Zahnrad 11 des Zahnradpaares 10 eingreift, wird das Zahnradpaar 10 um die Zahnradachse 14 gedreht. Die Drehbewegung des Zahnradpaares 10 überträgt sich vom Zahnrad 12 über die Zahnstange B 13, die mit dem Schieber 3 fest verbunden ist. Der Schieber 3 schließt den in Fig. 2 bezeichneten Weg S linear in gegenläufiger Richtung zur Zahnstange A 8. Diese Schließbewegung ist durch den Pfeil auf der Zahnstange B 13 gekennzeichnet.

## Patentansprüche

1. Chirurgisches Instrument mit einem Getriebe, bei welchem eine linear eingeschobene Zahnstange A (8) über ein Zahnrad A (11) und ein Zahnrad B (12) eine gegenläufige Linearbewegung eines Schiebers (3) verursacht, wodurch das Instrument geöffnet bzw. geschlossen wird, wobei eine Zahnstange B (13) fest mit dem Schieber (3) verbunden ist, **dadurch gekennzeichnet, dass** über einen Hebel (2) die Zahnstange A (8) linear in ein Griff-stück (1) eingeschoben oder daraus herausgezogen wird und dass das Zahnrad A (11) und das Zahnrad B (12) ein fest miteinander verbundenes Zahnradpaar (10) bilden, wobei das Gröβenverhältnis dieser beiden Zahnräder das Übersetzungsverhältnis bzw. das Untersetzungsverhältnis des Instrumentes bestimmt.

2. Instrument nach Anspruch 1 **dadurch gekennzeichnet, dass** das Zahnradpaar (10) drehbar im Griffstück (1) gelagert ist.

3. Instrument nach einem der vorangegangenen Ansprüche, **dadurch gekennzeichnet, dass** der Hebel (2) drehbar im Griffstück (1) gelagert ist und durch ein Federpaar (6/7) geöffnet wird.

4. Instrument nach einem der vorangegangenen Ansprüche, **dadurch gekennzeichnet, dass** ein Deckel (4) die Zahnstange A (8) und das Zahnradpaar (10) seitlich führt und in Eingriff hält.

5. Instrument nach einem der vorangegangenen Ansprüche, **dadurch gekennzeichnet, dass** eine Blende (5) die Zahnstange B (13) des Schiebers (3) vor Verschmutzung und Verletzungsgefahr schützt.

## Claims

1. A surgical instrument with a gear mechanism, in which a toothed rack A (8) inserted in a linear manner causes a linear movement of a slide (3) in the opposite direction by way of a toothed wheel A (11) and a toothed wheel B (12), as a result of which the instrument is opened or closed respectively, wherein a toothed rack B (13) is connected to the slide (3) in a fixed manner, **characterized in that** the toothed rack A (8) is inserted into a handle member (1) or is withdrawn therefrom in a linear manner by way of a lever (2), and the toothed wheel A (11) and the toothed wheel B (12) form a pair of toothed wheels (10) connected to each other in a fixed manner, wherein the size ratio of these two toothed wheels determines the step-up or step-down gear ratio of the instrument.

2. An instrument according to claim 1, **characterized in that** the pair of toothed wheels (10) are mounted in the handle member (1) in a rotatable manner.

3. An instrument according to one of the preceding claims, **characterized in that** the lever (2) is mounted in the handle member (1) in a rotatable manner and is opened by a pair of springs (6/7).

4. An instrument according to any one of the preceding claims, **characterized in that** a cover (4) guides the toothed rack A (8) and the pair of toothed wheels (10) laterally and keeps them engaged.

5. An instrument according to any one of the preceding claims, **characterized in that** a screen (5) protects the toothed rack B (13) of the slide (3) from dirt and the risk of damage.

## Revendications

1. Instrument chirurgical équipé d'un jeu d'engrenage, dans lequel une crémaillère A (8) mobile en translation linéaire provoque un mouvement linéaire en sens opposé d'un coulisseau (3) par l'intermédiaire d'une roue dentée A (11) et d'une roue dentée B (12), de façon à ouvrir ou à fermer l'instrument, une crémaillère B (13) étant fixée solidairement au coulisseau (3),
**caractérisé en ce que**
la crémaillère A (8) peut être poussée dans une poignée (1) ou extraite de celle-ci par translation linéaire au moyen d'un levier (2), et la roue dentée A (11) et la roue dentée B (12) forment une paire de roues dentées (10) fixées solidairement l'une à l'autre, le rapport de dimension de ces deux roues dentées déterminant le rapport de multiplication ou le rapport de démultiplication de l'instrument.

2. Instrument conforme à la revendication 1,
**caractérisé en ce que**
la paire de roues dentées (10) est montée mobile en rotation dans la poignée (1).

3. Instrument conforme à l'une des revendications précédentes,
**caractérisé en ce que**
le levier (2) est monté mobile en rotation dans la poignée (1) et peut être ouvert par une paire de ressort (6, 7).

4. Instrument conforme à l'une des revendications précédentes,
**caractérisé en que** qu'
un couvercle (4) guide latéralement la crémaillère A (8) et la paire de roues dentées (10) et les maintient en prise.

5. Instrument conforme à l'une des revendications précédentes,
**caractérisé en qu'**
un capot (5) protège la crémaillère B (13) du coulisseau (3) contre l'encrassement et le risque d'endommagement.
